# EUROPEAN PATENT SPECIFICATION

(11) **EP 3 020 290 B1**
(45) Date of publication and mention of the grant of the patent: **10.07.2024**
(21) Application number: 15192478.4
(22) Date of filing: 31.10.2015
(51) Int. Cl.: A61M 15/06, A24F 40/42, A24F 40/485, A61M 11/04, A61M 15/00

(54) **ATOMIZER AND ELECTRONIC CIGARETTE HAVING SAME**
ZERSTÄUBER UND ELEKTRONISCHE ZIGARETTE DAMIT
DISPOSITIF D'ATOMISATION ET CIGARETTE ÉLECTRONIQUE COMPORTANT CELUI-CI

(30) Priority: 13.11.2014 CN 201420673779 U
(43) Date of publication of application: 18.05.2016
(73) Proprietor: Shenzhen First Union Technology Co., Ltd., Shenzhen, Guangdong 518104 (CN)
(72) Inventor: LI, Yonghai, 518104 Shenzhen (CN); XU, Zhongli, 518104 Shenzhen (CN); ZHAO, Xiaoqiang, 518104 Shenzhen (CN)
(74) Representative: Proi World Intellectual Property GmbH

(56) References cited:
- EP-A1- 2 885 986
- WO-A1-2013/159245
- CN-A- 104 114 050
- US-A- 2 860 638

## Description

### TECHNICAL FIELD

The present invention relates to electronic cigarettes, and particularly to an atomizer and an electronic cigarette using same.

### BACKGROUND ART

**A** typical electronic cigarette includes a disposable liquid supply, an atomizing component, and a power supply. The disposable liquid supply is configured for storing tobacco liquid. The atomizing component is configured for generating aerosol from the tobacco liquid. The power supply is adapted for providing the atomizing component power. Generally, the disposable liquid supply is coupled to the atomizing component via screw threads. After the atomizing component is engaged with the disposable liquid supply, the liquid supply is pierced and the tobacco liquid flows out to the atomizing component for atomization. To avoid liquid leakage, a sealing element is arranged between the liquid supply and the atomizing component.

However, the atomizing component needs to be screwed several turns from a first position, where the liquid supply is first pierced, to a second position, where the liquid supply and the atomizing component are hermetically coupled. During this process, the tobacco liquid may leak via screw threads, rendering users experience unsatisfactory.

What is needed, therefore, are an atomizer and an electronic cigarette using same, which can overcome the above shortcomings.
CN 104114050 A discloses an electronic cigarette and an atomizer thereof. EP2885986 A1 discloses a capsule for use with an electronic smoking device. WO2013/159245 A1 discloses an electronic cigarette with sealed cartridge.

### SUMMARY

**An** exemplary atomizer includes a liquid supply and an atomizing component. The liquid supply is configured for storing tobacco liquid. The liquid supply has an open end and a sealing layer for sealing the open end. The atomizing component is detachably coupled to the liquid supply by snap fit. The atomizing component is configured for heating the tobacco liquid to vaporize. The atomizing component includes a piercing element, a buffer chamber, and an atomizing unit. The piercing element is configured for pricking the sealing layer, so that the tobacco liquid in the liquid chamber flows into the buffer chamber, when the atomizing component is engaged with the liquid supply. The buffer chamber is configured for storing tobacco liquid flowed from the liquid chamber. The atomizing unit is configured for absorbing the tobacco liquid from the buffer chamber, and generating aerosol from the tobacco liquid.
The present invention provides an atomizer according to independent claim 1.
Further improvements are recited in the dependent claims.

### BRIEF DESCRIPTION OF THE DRAWINGS

**Many** aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is a cross-sectional view of an atomizer according to a first embodiment, which forms no part of the invention.
FIG. 2 is an exploded view of the atomizer of FIG. 1.
FIG. 3 is a cross-sectional view of an atomizer according to a second embodiment.
FIG. 4 is an exploded view of the atomizer of FIG. 3.
FIG. 5 is a cross-sectional view of the atomizer of FIG. 3 after assembled.
FIG. 6 is a cross-sectional view of an atomizer according to a third embodiment, which forms no part of the invention.
FIG. 7 is a partially perspective view of an atomizing component in the atomizer of FIG. 6.
FIG. 8 is a side view of an electronic cigarette according to a fourth embodiment, which forms no part of the invention.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present disclosure.

The disclosure is illustrated by way of example and not by way of limitation in the figures of the accompanying drawings in which like references indicate similar elements. It should be noted that references to "an" or "one" embodiment in this disclosure are not necessarily to the same embodiment, and such references mean at least one.

Several definitions that apply throughout this disclosure will now be presented.

The term "outside" refers to a region that is beyond the outermost confines of a physical object. The term "inside" indicates that at least a portion of a region is partially contained within a boundary formed by the object. The term "substantially" is defined to be essentially conforming to the particular dimension, shape or other word that substantially modifies, such that the component need not be exact. For example, substantially cylindrical means that the object resembles a cylinder, but can have one or more deviations from a true cylinder. The term "comprising," when utilized, means "including, but not necessarily limited to"; it specifically indicates openended inclusion or membership in the so-described combination, group, series and the like.

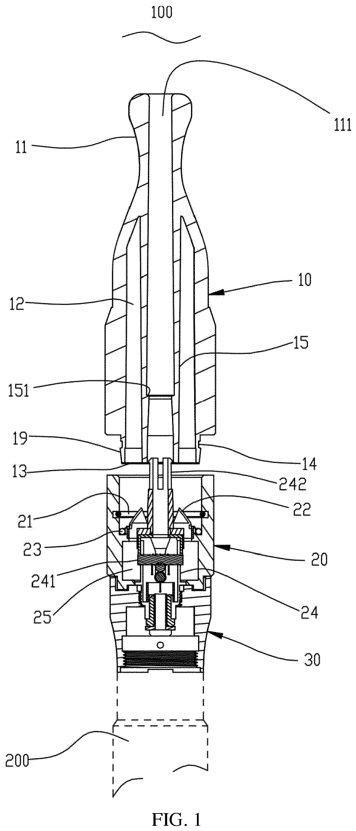

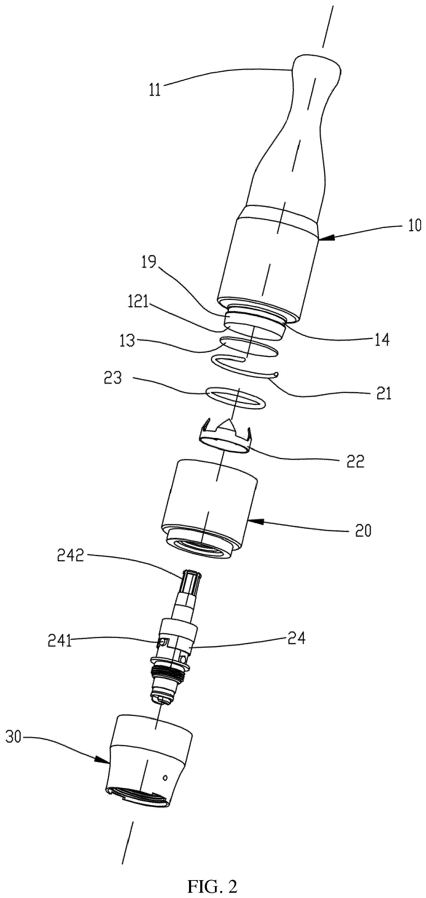

Referring to FIGS. 1-2, an atomizer 100 is shown. The atomizer 100 is configured (i.e., structured and arranged) for assembling with a power supply 200 to form an electronic cigarette. The atomizer 100 includes a liquid supply 10, a liquid chamber 12 in the liquid supply 10, and an atomizing component 20. The liquid chamber 12 is configured for receiving tobacco liquid. The atomizing component 20 is configured for generating aerosol from tobacco liquid. The liquid supply 10 includes an opening 121 at one end. A sealing layer 13 is provided at the opening 121, and configured for sealing the tobacco liquid. The atomizing component 20 includes a piercing element 22 adapted for piercing the sealing layer 13. In the present embodiment, the liquid supply 10 includes an air pipe 15 coaxially arranged with the liquid supply 10. The air pipe 15 and the liquid supply 10 are integrally formed, and made of an identical material. The liquid chamber 12 is cooperatively defined between a housing of the liquid supply 10 and the air pipe 15. The atomizing component 20 includes a buffer chamber 25, a sealing ring 23, and an atomizing head 24. The buffer chamber 25 is configured for storing tobacco liquid flowed from the liquid chamber 12. The atomizing head 24 is in communication with the air pipe 15. The atomizing head 24 includes an atomizing unit 241. The atomizing component 20 is detachably connected with the liquid supply 10 by snap fit. After the atomizing component 20 is engaged with the power supply 10, the piercing element 22 pricks the sealing layer 13, so that the tobacco liquid in the liquid chamber 12 flows into the buffer chamber 25 for atomization. In this position, an open end of the liquid supply 10 is tightly pressed against the sealing ring 13, thus avoiding liquid leakage.

The liquid supply 10 includes a mouthpiece 11 having an air outlet 111. The air outlet 111 is in communication with the air pipe 15. In the present embodiment, the mouthpiece 11 and the liquid supply 10 are integrally formed.

Further, the liquid supply 10 includes a plug end 19 adapted for inserting into the atomizing component 20. The atomizing component 20 includes a first clip ring 21 configured for coupling with the plug end 19. The plug end 19 defines a first groove 14 in an outer surface. The first clip ring 21 is engaged in the first groove 14 in use.

Further, the air pipe 15 includes a stepped portion 151 formed on an inner surface, and the atomizing head 24 includes a locking portion 242. After the atomizing component 20 is coupled to the liquid supply 10, an end of the atomizing head 24 is inserted into the air pipe 15, and the locking portion 242 is engaged in the stepped portion 151.

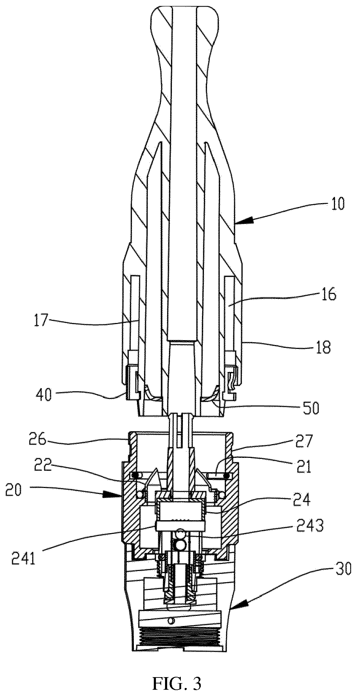

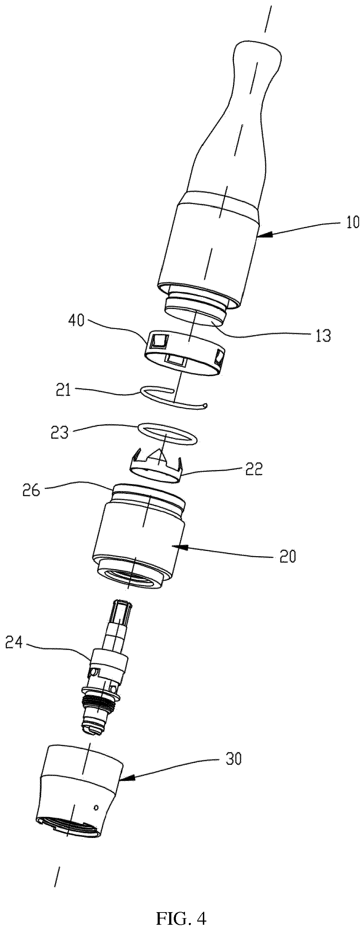

Referring to FIGS. 3-4, as an alternative embodiment, the atomizing component 20 is inserted into the liquid supply 10. The atomizing component 20 includes a connection end 26 for inserting into the liquid supply 10. Correspondingly, the liquid supply 10 includes a second clip ring 40 for locking the connection end 26, and the connection end 26 defines a second groove 27 in an exterior surface. The second clip ring 40 is engaged with the second groove 27. The liquid supply 10 includes an annular chamber 16 for connecting with the atomizing component 20. The annular chamber 16 divides the housing of the liquid supply 10 into a first housing 18 and a second housing 17. The second housing 17 is inserted into the atomizing component 20, and is clamped by the first clip ring 21. The connection end 26 is plugged into the annular chamber 16, and is clamped by the second clip ring 40.

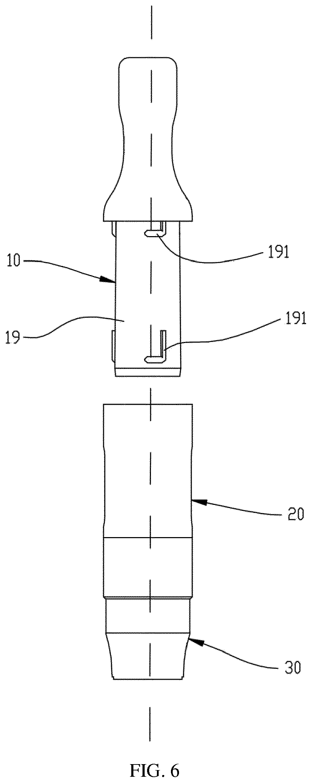

Referring to FIGS. 6-7, as an alternative embodiment, the liquid supply 10 includes a plug end 19 for inserting into the atomizing component 20. The plug end 19 includes a plurality of L-shaped protrusions 191 on an exterior surface, and correspondingly, the atomizing component 20 defines a plurality of L-shaped grooves 28 on an interior surface. The grooves 28 match with the protrusions 191. The liquid supply 10 is rotated a predetermined angle so that the protrusions 191 engage in the grooves 28. During assembly, after the plug end 19 is inserted into the atomizing component 20, the open end of the liquid supply 10 is tightly pressed against the sealing ring 13, liquid leakage will not occur during the rotation of the predetermined angle.

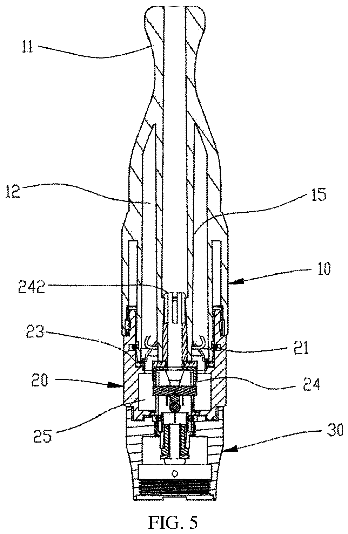

**To** further prevent liquid leakage, referring to FIGS. 3 and 5, the liquid supply 10 includes a check valve 50 in the open end. After the piercing element 22 pierces the sealing layer 13, the piercing element 22 pushes the check valve 50 to an open state, so that the tobacco liquid flows to the buffer chamber 25 via the check valve 50. In the present embodiment, the atomizing head 24 includes two atomizing units 241, and a liquid conducting element 243 between the two atomizing units 241. The liquid conducting element 243 is configured for guiding tobacco liquid, and preventing heating elements of the two atomizing units 241 from contacting with each other.

**In** use, the tobacco liquid flows from the liquid chamber 12 to the buffer chamber 25. The atomizing units 241 absorb the tobacco liquid in the buffer chamber 25, and heat the tobacco liquid to form aerosol. The aerosol is expelled from the air outlet 111 via the air pipe 15. In the present embodiment, the atomizing component 20 further includes a connection part 30 at an end for connecting with an external power supply.

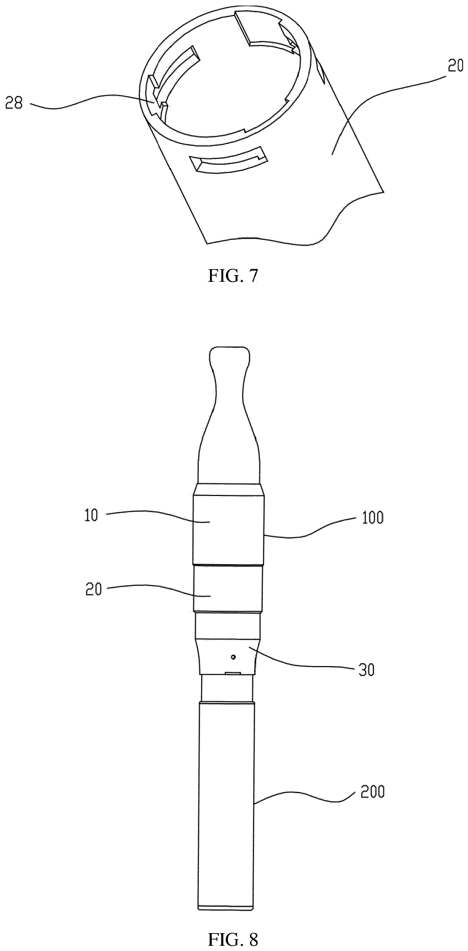

Referring to FIG. 8, an electronic cigarette includes an atomizer 100 and a power supply 200. The power supply 200 is engaged with the atomizer 100 via screw threads. The power supply 200 is configured for supplying the atomizer 100 power.

It is understood that the above-described embodiments are intended to illustrate rather than limit the disclosure. Variations may be made to the embodiments and methods.

## Claims

1. An atomizer (100) for an electronic cigarette, comprising:
a liquid supply (10) configured for storing tobacco liquid, the liquid supply (10) having an open end and a sealing layer (13) for sealing the open end; and
an atomizing component (20) detachably coupled to the liquid supply (10) by snap fit, the atomizing component (20) being configured for heating the tobacco liquid to vaporize, the atomizing component (20) comprising:
a piercing element (22) configured for pricking the sealing layer (13), so that the tobacco liquid in the liquid chamber (12) flows into a buffer chamber (25), when the atomizing component (20) is engaged with the liquid supply (10);
the buffer chamber (25) configured for storing tobacco liquid flowed from the liquid chamber (12); and
an atomizing unit configured for absorbing the tobacco liquid from the buffer chamber (25), and generating aerosol from the tobacco liquid;
**characterized in that** the liquid supply (10) further comprises a check valve (50) at the open end, and the piercing element (22) is configured for pushing the check valve (50) to an open state after the piercing element (22) pierces the sealing layer (13).

2. The atomizer in accordance with claim 1, wherein the liquid supply (10) comprises a plug end (19) adapted for inserting into the atomizing component (20), the plug end (19) defines a first groove (14) in an outer surface; the atomizing component (20) comprises a first clip ring (21), and the first clip ring (21) is engaged in the first groove (14).

3. The atomizer (100) in accordance with claim 1, wherein the atomizing component (20) comprises a connection end for inserting into the liquid supply (10); the liquid supply (10) comprises a second clip ring (40) for locking the connection end (26); the connection end (26) defines a second groove (27) in an exterior surface, and the second clip ring (40) is engaged with the second groove (27).

4. The atomizer (100) in accordance with claim 1, wherein the liquid supply (10) comprises a housing having an annular chamber (16), the annular chamber (16) divides the housing of the liquid supply (10) into a first housing (18) and a second housing (17); the atomizing component (20) further comprises a first clip ring (21), the liquid supply (10) further comprises a second clip ring (40) arranged in the annular chamber (16), the second housing (17) is inserted into the atomizing component (20), and is clamped by the first clip ring (21); the atomizing component (20) further comprises a connection end (26) plugged into the annular chamber (16), and the connection end (26) is clamped by the second clip ring (40).

5. The atomizer (100) in accordance with claim 1, wherein the liquid supply (10) further comprises an air pipe (15), the atomizing component (20) further comprises an atomizing head (24) in communication with the air pipe (15), and the atomizing unit (241) is arranged in the atomizing head (24).

6. The atomizer (100) in accordance with claim 4, wherein the air pipe (15) comprises a stepped portion (151) formed on an inner surface thereof, and the atomizing head (24) comprises a locking portion (242), an end of the atomizing head (24) is inserted into the air pipe (15), and the locking portion (242) is engaged in the stepped portion (151).

7. The atomizer (100) in accordance with claim 4, wherein the air pipe (15) and the liquid supply (10) are coaxially arranged, and the air pipe (15) and the liquid supply (10) are integrally formed.

8. The atomizer (100) in accordance with claim 4, wherein the liquid supply (10) further comprises a mouthpiece (11) defining an air outlet (111), the mouthpiece (11) and the liquid supply (10) are integrally formed, and the air outlet (111) is in communication with the air pipe (15).

9. The atomizer (100) in accordance with claim 4, wherein the atomizing head (24) comprises two atomizing units (241), and a liquid conducting element (243) sandwiched between the two atomizing units (241).

10. An electronic cigarette, comprising:
an atomizer (100) according to any of claims 1 - 9; and
a power supply (200) configured for supplying the atomizer (100) power.

## Patentansprüche

1. Ein Zerstäuber (100) für eine elektronische Zigarette, umfassend:
einen Flüssigkeitsvorrat (10), der zum Speichern von Tabakflüssigkeit konfiguriert ist, wobei der Flüssigkeitsvorrat (10) ein offenes Ende und eine Dichtungsschicht (13) zum Abdichten des offenen Endes aufweist; und
eine Zerstäubungskomponente (20), die abnehmbar mit der Flüssigkeitszufuhr (10) durch Schnappverbindung gekoppelt ist, wobei die Zerstäubungskomponente (20) zum Erhitzen der Tabakflüssigkeit zum Verdampfen konfiguriert ist, wobei die Zerstäubungskomponente (20) umfasst:
ein Durchstechelement (22), das so konfiguriert ist, dass es die Dichtungsschicht (13) durchsticht, so dass die Tabakflüssigkeit in der Flüssigkeitskammer (12) in eine Pufferkammer (25) fließt, wenn die Zerstäuberkomponente (20) mit der Flüssigkeitszufuhr (10) in Eingriff gebracht wird;
die Pufferkammer (25), die zum Speichern von Tabakflüssigkeit, die aus der Flüssigkeitskammer (12) fließt, konfiguriert ist; und
eine Zerstäubungseinheit, die so konfiguriert ist, dass sie die Tabakflüssigkeit aus der Pufferkammer (25) aufnimmt und aus der Tabakflüssigkeit ein Aerosol erzeugt;
**dadurch gekennzeichnet, dass** die Flüssigkeitszufuhr (10) ferner ein Rückschlagventil (50) am offenen Ende umfasst, und das Stechelement (22) so konfiguriert ist, dass es das Rückschlagventil (50) in einen offenen Zustand drückt, nachdem das Stechelement (22) die Dichtungsschicht (13) durchstochen hat.

2. Zerstäuber nach Anspruch 1, wobei die Flüssigkeitszufuhr (10) ein Stopfenende (19) aufweist, das zum Einführen in die Zerstäubungskomponente (20) geeignet ist, wobei das Stopfenende (19) eine erste Nut (14) in einer Außenfläche definiert; die Zerstäubungskomponente (20) einen ersten Klemmring (21) aufweist und der erste Klemmring (21) in die erste Nut (14) eingreift.

3. Zerstäuber (100) nach Anspruch 1, wobei die Zerstäuberkomponente (20) ein Anschlussende zum Einführen in die Flüssigkeitszufuhr (10) umfasst; die Flüssigkeitszufuhr (10) einen zweiten Klemmring (40) zum Verriegeln des Anschlussendes (26) umfasst; das Anschlussende (26) eine zweite Nut (27) in einer Außenfläche definiert und der zweite Klemmring (40) mit der zweiten Nut (27) in Eingriff steht.

4. Zerstäuber (100) nach Anspruch 1, wobei die Flüssigkeitszufuhr (10) ein Gehäuse mit einer Ringkammer (16) umfasst, die Ringkammer (16) das Gehäuse der Flüssigkeitszufuhr (10) in ein erstes Gehäuse (18) und ein zweites Gehäuse (17) unterteilt; die Zerstäubungskomponente (20) ferner einen ersten Klemmring (21) umfasst, die Flüssigkeitszufuhr (10) ferner einen zweiten Klemmring (40) umfasst, der in der Ringkammer (16) angeordnet ist, das zweite Gehäuse (17) in die Zerstäubungskomponente (20) eingesetzt ist und durch den ersten Klemmring (21) festgeklemmt wird; die Zerstäubungskomponente (20) ferner ein Anschlussende (26) umfasst, das in die Ringkammer (16) gesteckt ist, und das Anschlussende (26) durch den zweiten Klemmring (40) festgeklemmt wird.

5. Zerstäuber (100) nach Anspruch 1, wobei die Flüssigkeitszufuhr (10) ferner eine Luftleitung (15) umfasst, die Zerstäubungskomponente (20) ferner einen Zerstäubungskopf (24) umfasst, der mit der Luftleitung (15) in Verbindung steht, und die Zerstäubungseinheit (241) in dem Zerstäubungskopf (24) angeordnet ist.

6. Zerstäuber (100) nach Anspruch 4, wobei das Luftrohr (15) einen gestuften Abschnitt (151) aufweist, der an einer Innenfläche desselben ausgebildet ist, und der Zerstäuberkopf (24) einen Verriegelungsabschnitt (242) aufweist, wobei ein Ende des Zerstäuberkopfes (24) in das Luftrohr (15) eingeführt ist und der Verriegelungsabschnitt (242) in den gestuften Abschnitt (151) eingreift.

7. Zerstäuber (100) nach Anspruch 4, wobei das Luftrohr (15) und die Flüssigkeitszufuhr (10) koaxial angeordnet sind und das Luftrohr (15) und die Flüssigkeitszufuhr (10) einstückig ausgebildet sind.

8. Zerstäuber (100) nach Anspruch 4, wobei die Flüssigkeitszufuhr (10) ferner ein Mundstück (11) umfasst, das einen Luftauslass (111) definiert, das Mundstück (11) und die Flüssigkeitszufuhr (10) einstückig ausgebildet sind und der Luftauslass (111) in Verbindung mit dem Luftrohr (15) steht.

9. Zerstäuber (100) nach Anspruch 4, wobei der Zerstäuberkopf (24) zwei Zerstäubereinheiten (241) und ein flüssigkeitsleitendes Element (243) umfasst, das zwischen den beiden Zerstäubereinheiten (241) angeordnet ist.

10. Eine elektronische Zigarette, bestehend aus:
einen Zerstäuber (100) nach einem der Ansprüche 1 bis 9; und
eine Stromversorgung (200), die so konfiguriert ist, dass sie den Zerstäuber (100) mit Strom versorgt.

## Revendications

1. Un atomiseur (100) pour une cigarette électronique, comprenant :
une réserve de liquide (10) configurée pour stocker du tabac liquide, la réserve de liquide (10) ayant une extrémité ouverte et une couche d'étanchéité (13) pour sceller l'extrémité ouverte ; et
un composant d'atomisation (20) couplé de manière amovible à l'alimentation en liquide (10) par encliquetage, le composant d'atomisation (20) étant configuré pour chauffer le liquide du tabac afin de le vaporiser, le composant d'atomisation (20) comprenant :
un élément de perçage (22) configuré pour piquer la couche d'étanchéité (13), de sorte que le tabac liquide dans la chambre à liquide (12) s'écoule dans une chambre tampon (25), lorsque le composant d'atomisation (20) est en prise avec l'alimentation en liquide (10) ;
la chambre tampon (25) configurée pour stocker le tabac liquide s'écoulant de la chambre à liquide (12) ; et
une unité d'atomisation configurée pour absorber le liquide de tabac dans la chambre tampon (25) et générer un aérosol à partir du liquide de tabac ;
**caractérisé par le fait que** l'alimentation en liquide (10) comprend en outre un clapet anti-retour (50) à l'extrémité ouverte, et que l'élément de perçage (22) est configuré pour pousser le clapet anti-retour (50) à l'état ouvert après que l'élément de perçage (22) a percé la couche d'étanchéité (13).

2. L'atomiseur selon la revendication 1, dans lequel l'alimentation en liquide (10) comprend une extrémité de bouchon (19) adaptée pour être insérée dans le composant d'atomisation (20), l'extrémité de bouchon (19) définit une première rainure (14) dans une surface extérieure ; le composant d'atomisation (20) comprend une première bague d'agrafe (21), et la première bague d'agrafe (21) est engagée dans la première rainure (14).

3. L'atomiseur (100) selon la revendication 1, dans lequel le composant d'atomisation (20) comprend une extrémité de connexion à insérer dans l'alimentation en liquide (10) ; l'alimentation en liquide (10) comprend une deuxième bague de serrage (40) pour verrouiller l'extrémité de connexion (26) ; l'extrémité de connexion (26) définit une deuxième rainure (27) dans une surface extérieure, et la deuxième bague de serrage (40) est engagée dans la deuxième rainure (27).

4. L'atomiseur (100) selon la revendication 1, dans lequel l'alimentation en liquide (10) comprend un boîtier ayant une chambre annulaire (16), la chambre annulaire (16) divise le boîtier de l'alimentation en liquide (10) en un premier boîtier (18) et un second boîtier (17) ; le composant d'atomisation (20) comprend en outre une première bague de serrage (21), l'alimentation en liquide (10) comprend en outre une deuxième bague de serrage (40) disposée dans la chambre annulaire (16), le deuxième boîtier (17) est inséré dans le composant d'atomisation (20) et est serré par la première bague de serrage (21) ; le composant d'atomisation (20) comprend en outre une extrémité de connexion (26) enfichée dans la chambre annulaire (16), et l'extrémité de connexion (26) est serrée par la deuxième bague de serrage (40).

5. L'atomiseur (100) selon la revendication 1, dans lequel l'alimentation en liquide (10) comprend en outre un tuyau d'air (15), le composant d'atomisation (20) comprend en outre une tête d'atomisation (24) en communication avec le tuyau d'air (15), et l'unité d'atomisation (241) est disposée dans la tête d'atomisation (24).

6. Pulvérisateur (100) selon la revendication 4, dans lequel le tuyau d'air (15) comprend une partie étagée (151) formée sur sa surface intérieure, et la tête d'atomisation (24) comprend une partie de verrouillage (242), une extrémité de la tête d'atomisation (24) est insérée dans le tuyau d'air (15), et la partie de verrouillage (242) est engagée dans la partie étagée (151).

7. Pulvérisateur (100) selon la revendication 4, dans lequel le tuyau d'air (15) et l'alimentation en liquide (10) sont disposés coaxialement, et le tuyau d'air (15) et l'alimentation en liquide (10) sont formés d'un seul tenant.

8. L'atomiseur (100) selon la revendication 4, dans lequel l'alimentation en liquide (10) comprend en outre un embout (11) définissant une sortie d'air (111), l'embout (11) et l'alimentation en liquide (10) sont formés d'un seul tenant, et la sortie d'air (111) est en communication avec le tuyau d'air (15).

9. L'atomiseur (100) selon la revendication 4, dans lequel la tête d'atomisation (24) comprend deux unités d'atomisation (241), et un élément conducteur de liquide (243) pris en sandwich entre les deux unités d'atomisation (241).

10. Cigarette électronique comprenant
un atomiseur (100) selon l'une des revendications 1 à 9 ; et
une alimentation électrique (200) configurée pour alimenter l'atomiseur (100).
